(19) Europäisches Patentamt
European Patent Office
Office européen des brevets

(11) Veröffentlichungsnummer: **0 019 302**
**A1**

(12) # EUROPÄISCHE PATENTANMELDUNG

(21) Anmeldenummer: 80102827.5

(22) Anmeldetag: 21.05.80

(51) Int. Cl.³: C 07 C 69/95
C 07 H 15/24, C 12 P 19/56
C 12 P 29/00, A 61 K 31/71

(30) Priorität: 22.05.79 GB 7917763

(43) Veröffentlichungstag der Anmeldung:
26.11.80 Patentblatt 80/24

(84) Benannte Vertragsstaaten:
AT BE CH DE FR GB IT LI LU NL SE

(71) Anmelder: F. HOFFMANN-LA ROCHE & CO.
Aktiengesellschaft

CH-4002 Basel(CH)

(72) Erfinder: Fujiwara, Akiko
1059-7, Fueta
Kamakura-shi, Kanagawa-ken(JP)

(72) Erfinder: Hoshino, Tatsuo
808-47, Fueta
Kamakura-shi, Kanagawa-ken(JP)

(72) Erfinder: Tazoe, Masaaki
4-1-3-1008, Yokodai
Isogo-ku, Yokohama-shi, Kanagawa-ken(JP)

(74) Vertreter: Wuesthoff, Franz, Dr.-Ing. et al,
Patentanwälte Wuesthoff -v. Pechmann-Behrens-Goetz
Schweigerstrasse 2
D-8000 München 90(DE)

(54) **Neue tetracyclische Verbindungen, Verfahren zu deren Herstellung, deren Verwendung und pharmazeutische Präparate.**

(57) Verbindungen der allgemeinen Formel

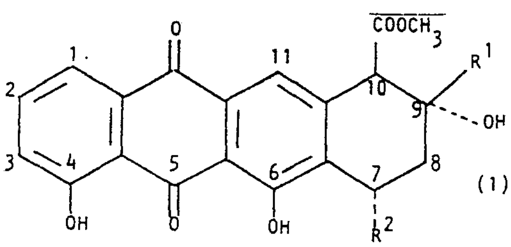

(1)

in der R¹ eine Methyl- oder Acetonylgruppe und R² ein Wasserstoffatom, eine Hydroxylgruppe oder eine Gruppe der Formel

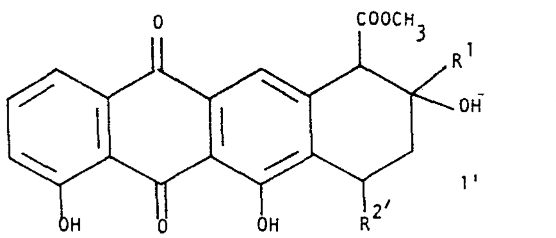

bedeuten,
ein Verfahren zur Herstellung dieser Verbindungen, bei dem man

(a) einen Mikroorganismus der Art Streptomyces galilaeus, der imstande ist, die Verbindungen der Formel I zu bilden, in wäßrigem Nährmedium unter aeroben Bedingungen züchtet und die Verbindungen aus der Fermentationsbrühe gewinnt;

(b) eine Verbindung der Formel

./...

EP 0 019 302 A1

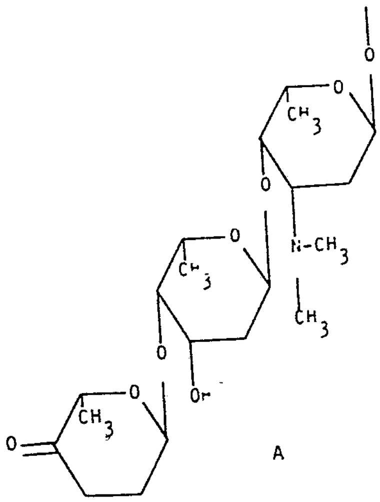

A

Oder

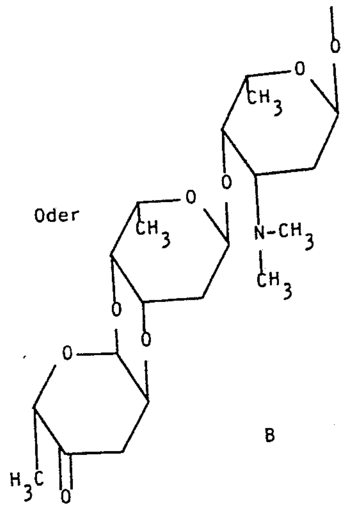

B

in der $R^1$ die oben angegebene Bedeutung hat und $R^{2'}$ eine Gruppe

der Formel A oder B bedeutet, unter sauren Bedingungen hydrolysiert, um die Gruppe $R^{2'}$ in eine Hydroxylgruppe umzuwandeln oder

(c) die Zuckergruppe einer Verbindung der Formel I' mit Hilfe eines Katalysators oder eines Enzyms abspaltet, um die Gruppe $R^{2'}$ der Formel I' durch ein Wasserstoffatom zu ersetzen,

und die Verwendung dieser Verbindungen als Zwischenprodukte für die Herstellung von Anthracyclin-Antibiotika oder als Wirkstoffe mit antibakterieller oder Antitumor-Aktivität.

Neue tetracyclische Verbindungen, Verfahren zu deren Herstellung, deren Verwendung und pharmazeutische Präparate.

Die Erfindung betrifft neue tetracyclische Verbindungen, ein Verfahren zu deren Herstellung, pharmazeutische Zubereitungen bzw. Arzneimittel, die diese Verbindungen enthalten und die gegen Tumoren wirksam sind, sowie neue Mikroorganismen, die zur Herstellung der Verbindungen angewandt werden.

Insbesondere betrifft die vorliegende Erfindung neue Anthracyclinone und deren Derivate entsprechend der allgemeinen Formel:

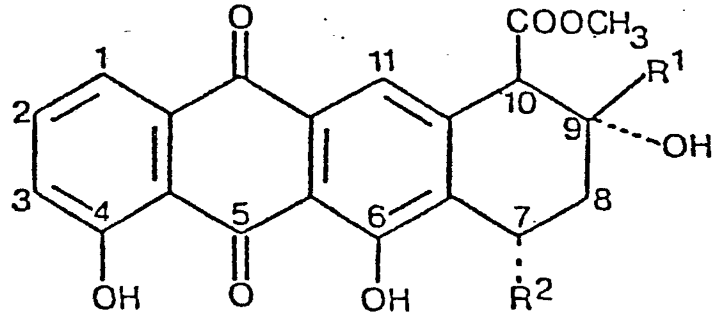

I,

Mez/12.5.80

in der $R^1$ eine Methyl- oder Acetonylgruppe, $R^2$ ein Wasserstoffatom, eine Hydroxylgruppe oder eine Gruppe der Formel

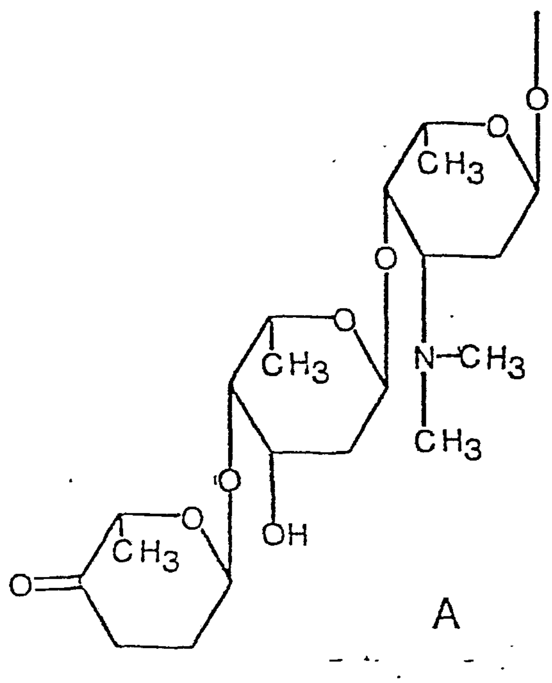

A

oder

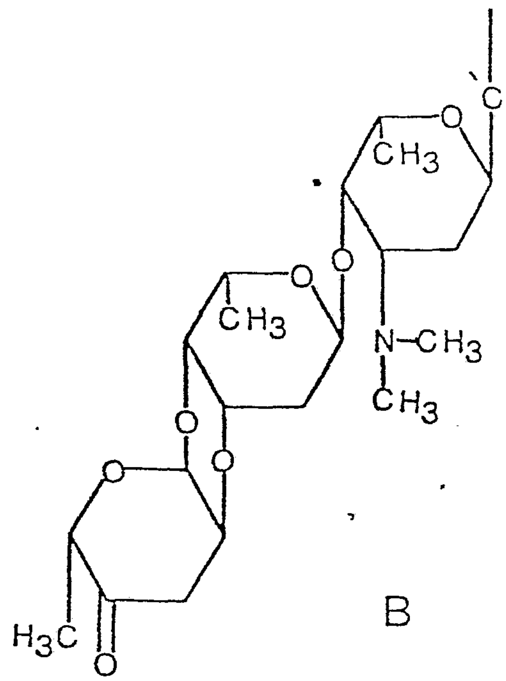

B

bedeutet.

In dieser Beschreibung und den Ansprüchen werden die speziellen Verbindungen, die unter die Formel I fallen, folgendermaßen bezeichnet:

| $R^1$ | $R^2$ | Verbindung |
|---|---|---|
| $-CH_3$ | H | 7-Desoxyauramycinon |
| | OH | Auramycinon |
| | A | Auramycin A |
| | B | Auramacin B |
| $-CH_2-CO-CH_3$ | H | 7-Desoxysulfurmycinon |
| | OH | Sulfurmycinon |
| | A | Sulfurmycin A |
| | B | Sulfurmycin B |

Die neuen erfindungsgemäßen Verbindungen sind gekennzeichnet durch die folgenden physikalisch-chemischen Daten:

[ Die bei der Dünnschichtchromatographie (TLC) angewandten Lösungsmittel sind Chloroform/Methanol 10:1 (Volumen) (Lösungsmittel A); Chloroform/Methanol 100:1 (Volumen) (Lösungsmittel B); Toluol/Methanol 10:1 (Volumen) (Lösungsmittel C) und Toluol/Methanol 100:1 (Volumen) (Lösungsmittel D) ] :

<u>Auramycin A</u> ($C_{41}H_{51}O_{15}N$)

MG: 797,3
Fp.: 141°C (Zers.)
Spezifische Drehung: $[\alpha]_D^{20}$ = -8,0° (c = 0,1 in Chloroform)
TLC (Silicagel): $R_f$ 0,45 (Lösungsmittel A)
$R_f$ 0,21 (Lösungsmittel C)

<u>Auramycin B</u> ($C_{41}H_{49}O_{15}N$)

MG: 795,3
Fp.: 161°C (Zers.)
Spezifische Drehung: $[\alpha]_D^{20}$ = -8,0° (c = 0,1 in Chloroform)
TLC (Silicagel): $R_f$ 0,66 (Lösungsmittel A)
$R_f$ 0,43 (Lösungsmittel C)

<u>Sulfurmycin A</u> ($C_{43}H_{53}O_{16}N$)

MG: 839,3
Fp.: 140°C (Zers.)
Spezifische Drehung: $[\alpha]_D^{20}$ = -23,3° (c = 0,1 in Chloroform)
TLC (Silicagel): $R_f$ 0,39 (Lösungsmittel A)
$R_f$ 0,15 (Lösungsmittel C)

<u>Sulfurmycin B</u> ($C_{43}H_{51}O_{16}N$)

MG: 837,3
Fp.: 149°C (Zers.)
Spezifische Drehung: $[\alpha]_D^{20}$ = -21,5° (c = 0,1 in Chloroform)
TLC (Silicagel): $R_f$ 0,61 (Lösungsmittel A)
$R_f$ 0,38 (Lösungsmittel C)

<u>Auramacinon</u> ($C_{21}H_{18}O_8$)

MG: 398,1
Fp.: 153,5°C
Spezifische Drehung: $[\alpha]_D^{20}$ = + 178,0° (c = 0,1 in Chloroform)
TLC (Silicagel): $R_f$ 0,24 (Lösungsmittel B)
$R_f$ 0,32 (Lösungsmittel D)

<u>Sulfurmycinon</u> ($C_{23}H_{20}O_9$)

MG: 440,1
Fp.: 159°C
Spezifische Drehung: $[\alpha]_D^{20}$ = +232,2° (c = 0,1 in Chloroform)
TLC (Silicagel): $R_f$ 0,28 (Lösungsmittel B)
$R_f$ 0,28 (Lösungsmittel D)

<u>7-Desoxyauramycinon</u> ($C_{21}H_{18}O_7$)

MG: 382,1
Fp.: 200°C
Spezifische Drehung: $[\alpha]_D^{20}$ = +81,2° (c = 0,1 in Chloroform)
TLC (Silicagel): $R_f$ 0,32 (Lösungsmittel B)
$R_f$ 0,44 (Lösungsmittel D)

<u>7-Desoxysulfurmycinon</u> ($C_{23}H_{20}O_8$)

MG: 424,1
Fp.: 219,5°C
Spezifische Drehung: $[\alpha]_D^{20}$ = +73,9° (c = 0,1 in Chloroform)

TLC (Silicagel): $R_f$ 0,49 (Lösungsmittel B)
$R_f$ 0,60 (Lösungsmittel D)

Gemäß dem erfindungsgemäßen Verfahren werden die neuen Verbindungen der Formel I hergestellt, indem man

(a) einen Mikroorganismus der Art Streptomyces galilaeus, der imstande ist, die Verbindungen der Formel I in einem wäßrigen Medium unter aeroben Bedingungen zu bilden, züchtet und die Verbindungen aus der Fermentationsbrühe gewinnt;

(b) unter sauren Bedingungen eine Verbindung der allgemeinen Formel:

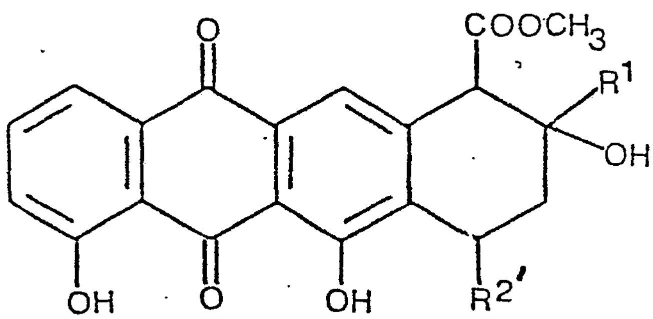

I' ,

in der $R^1$ die oben angegebene Bedeutung hat und $R^{2'}$ eine Gruppe der Formel A oder B bedeutet, hydrolysiert, um die Gruppe $R^{2'}$ in eine Hydroxylgruppe umzuwandeln oder

(c) den Zuckerrest von einer Verbindung der Formel I' mit Hilfe eines Katalysators oder eines Enzyms abspaltet, um die Gruppe $R^{2'}$ in der Formel I' durch ein Wasserstoffatom zu ersetzen.

Der in der Stufe (a) des oben angegebenen Verfahrens angewandte Mikroorganismus umfaßt alle Stämme, die zu der Art Streptomyces galilaeus gehören und die imstande sind, die Verbindungen der Formel I zu erzeugen, einschließlich der Mutanten und Varianten. Bevorzugte Stämme sind Streptomyces

galilaeus OBB-111 und Streptomyces galilaeus FR-401, die aus Bodenproben in Neuschwanstein und Murnau/Oberbayern (Westdeutschland) isoliert worden sind, sowie deren Mutanten und Varianten, vorzugsweise Streptomyces galilaeus OBB-111-610, der erhalten worden ist durch Behandlung von Streptomyces galilaeus OBB-111 mit N-Methyl-N'-nitro-N-nitrosoguanidin. Solche Mutanten können von Ausgangsformen (Elternformen) durch übliche Mutationsverfahren erhalten werden; z.B. durch Bestrahlung mit UV-Licht, Röntgenstrahlen oder γ-Strahlen oder durch Behandlung mit geeigneten Mutagenen. Die Stämme Streptomyces galilaeus OBB-111, Streptomyces galilaeus OBB-111-610 und Streptomyces galilaeus FR-401, die ebenfalls unter den Rahmen der Erfindung fallen, sind bei der Agency of Industrial Science and Technology, Fermentation Research Institute, Japan, unter den Nummern FERM-P Nr. 4780 (29. Januar 1979), FERM-P Nr. 4883 (22. März 1979) bzw. FERM-P Nr. 4882 (22. März 1979) hinterlegt worden sowie bei der American Type Culture Collection, Rockville, Maryland, USA unter den Nummern ATCC 31533, 31534 bzw. 31535.

Die mykologischen Charakteristika für diese Stämme sind:

1. <u>Morphologische Eigenschaften:</u>

Der Stamm OBB-111 (FERM-P Nr. 4780, ATCC 31533) bildet aus dem Substrat Mycel ein mäßig langes Luftmycel. An der Spitze des Luftmycels wird die Bildung von Haken oder Spiralen beobachtet; es tritt jedoch keine Wirtel-Bildung auf.

Üblicherweise werden reife Sporenketten mit mehr als 10 Sporen pro Kette gebildet. Die Sporen sind zylindrisch, messen 0,5 bis 0,6 µm x 0,8 bis 1,0 µm und ihre Oberfläche ist glatt.

Der Stamm FR-401 (FERM-P Nr. 4882, ATCC 31535) bildet aus dem Substrat-Mycel ein Luftmycel, das büschelartig verzweigt ist. Es wird die Bildung von Spiralen beobachtet, es werden jedoch keine Wirtel gebildet. Üblicherweise werden reife Sporenketten mit mehr als zehn Sporen pro Kette gebildet. Die Sporen sind zylindrisch oder ellipsoid und messen 0,6 bis 0,8 µm x 0,8 bis 1,2 µm und ihre Oberfläche ist glatt.

2. Kulturcharakteristika auf verschiedenen Medien:

Die Kulturcharakteristika der Stämme OBB-111 und FR-401 sind in der folgenden Tabelle 1 angegeben:

Die Farbe der Kolonien der Stämme OBB-111 und FR-401 auf Sacharose-Nitrat-Agar, Glycerin-Asparagin-Agar, Stärkeanorganische Salze-Agar und Hafermehl-Agar verändert sich von rosa nach violett bei tropfenweiser Zugabe von 0,05 n Natriumhydroxidlösung.

## Tabelle 1

Kulturcharakteristika der Stämme OBB-111 und FR-401

| Medium | Stamm OBB-111 | FR-401 |
|---|---|---|
| Saccharose-Nitrat-Agar | | |
| Kolonie | matt orange (4pe*), Orange-rost) | braun (6ni*), Taupe-braun) ~ blaß-rötlich orange (6ie, Rotholz) |
| Luftmycel | bräunlich-grau (3cb, Sand) ~ blaß-orange (5cb) | schwach-rosa-weiß (6ge, Rose-Gray) |
| diffundierbares Pigment | rötlich | rötlich |
| Glukose-Asparagin-Agar | | |
| Kolonie | blaß-orange (3pe, Topas ~ 3ne, Topas) | blaß-orange (4ge, Rose-beige) |
| Luftmycel | leicht bräunlich-grau (3dc, Natur) | keines |
| diffundierbares Pigment | bräunlich | bräunlich |
| Glycerin-Asparagin-Agar (ISP Medium Nr. 5) | | |
| Kolonie | blaß-gelb (3gc, hell-lohfarben) ~ blaß-gelblich-braun (3lc, Bernstein) | matt rötlich-orange (5le, Ruet-lohfarben ~ 5ne Ziegelrot) |
| Luftmycel | hellgrau (2fe, gedecktes Grau) | hellgrau (2fe, gedecktes Grau) |
| diffundierbares Pigment | keines | braun |

Fortsetzung Tabelle 1:

*) Diese Farbbezeichnungen folgen dem Farb-Standard des "Color Harmony Manual", publiziert von Container Corporated of America, USA.

Fortsetzung zu **Tabelle 1**

| Medium | Stamm OBB-111 | FR-401 |
|---|---|---|
| Stärke-anorganische Salze-Agar (ISP Medium Nr. 4) | | |
| Kolonie | blaßgelb (2pc, leuchtendes Gold ~matt-gelb (2pe, Senf-Gold) | grau-gelb (3ec, Bisquit) ~blaß-gelbliches Braun (3ge, Lt-lohfarben) |
| Luftmycel | hellbräunlich-grau (2dc, Natur) ~hellgrau (2fe, gedecktes Grau) | hellbräunlich-grau (3dc, Natur) |
| diffundierbares Pigment | gelb | bräunlich |
| Tyrosin-Agar (ISP Medium Nr. 7) | | |
| Kolonie | mittel-dunkelbräunlich-grau (3ni, Nelkenbraun) | bräunlich-violett (4pe, Dk Gewürzbraun) |
| Luftmycel | keines | hellgrau (3fe, Silbergrau) |
| diffundierbares Pigment | schwarz | braun |
| Nährstoff-Agar | | |
| Kolonie | farblos blaß-braun | farblos blaß-braun |
| Luftmycel | keines | keines |
| diffundierbares Pigment | keines | keines |
| Hefeextrakt-Malzextrakt-Agar (ISP Medium Nr. 2) | | |
| Kolonie | gelblich-braun (3ng, gelber Ahorn) | blaß-gelblich-braun (3ie, Kamel) |
| Luftmycel | hellgrau (2fe, gedecktes Grau) | hellgrau (2fe gedecktes Grau)~ hell-bräunlich grau (3dc, Natur) |
| diffundierbares Pigment | keines | keines |

Fortsetzung zu Tabelle 1

| Medium | Stamm OBB-111 | FR-401 |
|---|---|---|
| Hafermehl-Agar (ISP Medium Nr.3) | | |
| Kolonie | blaßgelblich-braun (2gc, Bambus) ~ blaß-braun (3ie, Kamel) | blaßgelblich-braun (3ie, Kamel) ~ blaß-rötlich-braun (4ge, Haut) |
| Luftmycel | hellgrau (2fe, gedecktes Grau ~ 3fe Silbergrau) | hellgrau bis rötlichbraun (5fe, Asche) |
| diffundierbares Pigment | braun | rot |
| Magermilch (37°C) | | |
| Kolonie | braun ~ dunkelbraun | braun ~ dunkelbraun |
| Luftmycel | weiß ~ bräunlich-grau | keines |
| diffundierbares Pigment | dunkelbraun | dunkelbraun |
| Glukose-Pepton-Gelatine-Stichkultur | | |
| Kolonie | blaßgelb | farblos |
| Luftmycel | keines | keines |
| diffundierbares Pigment | braun | braun |

3. Physiologische Eigenschaften:

Die physiologischen Eigenschaften sowie die Kohlenhydratverwertung der Stämme OBB-111 bzw. FR-401 sind in der folgenden Tabelle 2 bzw. 3 angegeben. Die Wachstumstemperatur wurde auf Hefeextrakt-Malzextrakt-Agar (ISP Medium Nr.2) bei 5, 20, 27, 32, 37, 45 und 55°C untersucht. Die optimale Temperatur für das Wachstum liegt bei 27 bis 32°C und bei 5, 45 und 55°C tritt kein Wachstum auf.

Tabelle 2

Physiologische Eigenschaften der Stämme OBB-111 und FR-401

| Versuch | Reaktion | angewandte Verfahren und Materialien |
|---|---|---|
| Gelatineverflüssigung | mäßige Verflüssigung | Glukose-Pepton-Gelatine-Medium, 27°C |
| Stärke-Hydrolyse | schwache bis mäßige Hydrolyse | Stärke-anorganische Salze-Agar |
| Peptonisierung und Koagulation von Magermilch | mäßige bis starke Peptonisierung und keine Koagulation | 10 % Magermilch, 37°C |
| Nitrat-Reduktion | positiv | ISP Medium Nr. 8;27°C |
| Melaninbildung | positiv | ISP Medium Nr. 1<br>ISP Medium Nr. 6<br>ISP Medium Nr. 7 |

## Tabelle 3

Kohlenhydratverwertung der Stämme OBB-111 und FR-401

| | |
|---|---|
| L-Arabinose | positiv |
| D-Xylose | positiv |
| Glukose | positiv |
| D-Fructose | positiv |
| Saccharose | positiv |
| Inositiol | positiv |
| L-Rhamnose | positiv |
| Raffinose | positiv |
| D-Mannit | negativ |

Grundmedium: Pridham-Gottlieb-Medium (ISP Medium Nr. 9)
Temperatur: 27°C.

Die oben angegebenen Eigenschaften der Stämme OBB-111 und FR-401 können folgendermaßen zusammengefaßt werden: Die Stämme gehören zu der Gattung Streptomyces. Das Luftmycel bildet am Ende Spiralen, aber keine Wirtel. Es zeigt sich, daß das Wachstum auf verschiedenen Medien blaß-gelblich-braun bis blaß-braun oder matt-orange ist und das Luftmycel hellgrau. Die Stämme bilden ein rötliches bis braunes diffundierbares Pigment und Melanin auf verschiedenen Medien. Die Stämme OBB-111 und FR-401 erinnern an die bekannten Stämme Streptomyces galilaeus (1: Archiv für Mikrobiologie, 31, 356, 1958. 2: The Actinomycetes, 2, 215, 1961. 3: International Journal of Systematic Bacteriology, 22, 298, 1972) und Streptomyces galilaeus MA 144-M1, FERM-P Nr. 2455 (1: JA-AS 34915/1976). Die Unterschiede zwischen den erfindungsgemäßen Stämmen und den Standardstämmen von S. galilaeus ISP 5481 und S. galilaeus MA 144-M1 (FERM-P Nr. 2455) wurden in parallelen Kulturen untersucht. Die Ergebnisse sind in der folgenden Tabelle 4 angegeben.

Tabelle 4

| | OBB-111 und FR-401 | S.galilaeus ISP 5481 | S.galilaeus MA 144-M1 (FERM-P Nr. 2455) |
| --- | --- | --- | --- |
| Verflüssigung von Gelatine | mäßig | schwach bis mäßig | schwach bis mäßig |
| Koagulation von Milch | negativ | schwach positiv | negativ |
| diffundierbares Pigment | dunkelbraun | hellbraun | dunkelbraun |
| Farbänderung des Wachstums durch 0,05 n Natriumhydroxidlösung: | | | |
| ISP Medium Nr. 3 | rosa bis violett | --- | rosa bis violett |
| ISP Medium Nr. 4 | leicht rosa bis violett | --- | leicht rosa bis violett |
| ISP Medium Nr. 5 | violett | leicht violett | violett |

Aus diesen Ergebnissen geht hervor, daß die erfindungsgemäßen Stämme OBB-111 und FR-401 sich von S. galilaeus MA 144-M1 (FERM-P Nr. 2455) bezüglich der Verflüssigung von Gelatine und von S. galilaeus ISP 5481 bezüglich der Koagulation von Magermilch, der Bildung von diffundierbarem Pigment und der Farbänderung des Wachstums durch 0,05n Natriumhydroxidlösung unterscheiden. Die erfindungsgemäßen Stämme sind jedoch S.galilaeus ISP 5481 und S. galilaeus MA 144-M1 (FERM-P Nr. 2455) bezüglich der Morphologie und Farbe des Wachstums und Mycels auf verschiedenen Medien der Chromogenität und Verwertung von Kohlenhydraten sehr ähnlich.

Jedoch können weder S. galilaeus ISP 5481 noch S. galilaeus MA 144-M1 (FERM-P Nr. 2455) die Verbindungen der Formel I erzeugen.

Gemäß einer bevorzugten Ausführungsform des erfindungsgemäßen Verfahrens (a) können die Verbindungen der Formel I gebildet werden durch Züchtung von Streptomyces galilaeus OBB-111, Streptomyces galilaeus OBB-111-610 (FERM-P Nr.4883, ATCC 31534) oder Streptomyces galilaeus FR-401 in einem wäßrigen Nährmedium unter aeroben Bedingungen.

Die Züchtung kann in einem Kulturmedium, enthaltend die üblichen Nährstoffe, durchgeführt werden. Als Kohlenstoffquellen dienen z.B. Glucose, Saccharose, Stärke, Lactose, Maltose , Fructose, Glycerin, Dextrin oder deren Gemische und als Stickstoffquellen z.B. Sojabohnenmehl, Baumwollsamenmehl, Fleischextrakt, Fischmehl, Pepton, Trockenhefe, Maiswasser, vorzugsweise Weizenkeime oder deren Gemische. Ferner kann das Kulturmedium, soweit erforderlich, geeignete anorganische Substanzen, wie Phosphate, Sulfate, Chloride, Bromide, Nitrate und Carbonate von Natrium, Kalium, Ammonium, Calcium u.ä. enthalten.

Die Züchtung kann in einem wäßrigen Medium unter aeroben Bedingungen, besonders als Submers-Kultur durchgeführt werden. Die bevorzugte Temperatur für die Züchtung liegt im Bereich von 20 bis 37°C, insbesondere 25 bis 30°C. Der pH-Wert des Mediums kann variieren, liegt jedoch im allgemeinen im Bereich von 5 bis 8.

Nach ungefähr 2 bis 10 Tage langer Züchtung unter den oben angegebenen Bedingungen können die Verbindungen der Formel I aus der Fermentationsbrühe gewonnen werden. Die so erhaltenen Verbindungen der Formel I, d.h. Auramycin A und B, Sulfurmycin A und B, Auramycinon, Sulfurmycinon, 7-Desoxyauramycinon und 7-Desoxysulfurmycinon können aus der Fermentationsbrühe gewonnen werden z.B. durch Extraktion mit einem mit Wasser nicht mischbaren organischen Lösungsmittel, wie Äthylacetat, Chloroform, Methylenchlorid, Methylisobutylketon oder einem Gemisch aus Chloroform und Methanol, vorzugsweise mit Chloroform/Methanol (1:1, Volumen). Die organische Phase wird abgetrennt und zu einem öligen Material getrocknet. Ein nicht-polares organisches Lösungsmittel, wie n-Hexan, wird zu diesem öligen Material zugegeben, wodurch die rohen Verbindungen als Pulver erhalten werden.

Die so erhaltenen Verbindungen können voneinander getrennt werden durch Chromatographie über Säulen, die mit einem Adsorbens, wie Silicagel, oder mit einem Dextrangel, wie Sephadex LH-20 u.ä. gepackt sind. Die Fraktionen werden durch Dünnschichtchromatographie und/oder Hochdruck-Flüssigkeits-Chromatographie analysiert und die entsprechenden Fraktionen zusammengegeben und eingedampft, wobei man die Verbindung in mehr oder weniger reiner Form erhält. Eine weitere Reinigung kann durch wiederholte Säulenchromatographie und/oder Hochdruck-Flüssigkeits-Chromatographie erreicht werden.

Die saure Hydrolyse von Auramycin A oder B oder von Sulfurmycin A oder B, entsprechend Stufe (b) des oben angegebenen Verfahrens, kann in an sich bekannter Weise durchgeführt werden mit Hilfe einer Säure, wie Salzsäure, Schwefelsäure, Phosphorsäure oder ähnlichem (0,1 bis 3 n). Die Hydrolyse kann bei einer Temperatur von 0°C bis zur Rückflußtemperatur des Hydrolysegemisches, vorzugsweise bei erhöhter Temperatur, durchgeführt werden. Durch diese saure Hydrolyse werden Auramycinon bzw. Sulfurmycinon erhalten.

Die Abspaltung der Zuckergruppe von einer Verbindung der Formel I', entsprechend Stufe (c) des oben angegebenen Verfahrens, kann ebenfalls in an sich bekannter Weise durchgeführt werden unter Verwendung eines Katalysators, wie Palladium, Platin, Rhodium, aktiviertem Nickel u.ä. oder einem

Enzym, das erhalten worden ist, z.B. aus Rattenleberhomogenisaten oder Zellen von Anthracyclin-Antibioticabildenden Mikroorganismen. 7-Desoxyauramycinon bzw. 7-Desoxysulfurmycinon werden nach dieser Arbeitsweise erhalten.

Die nach dem erfindungsgemäßen Verfahren hergestellten Anthracyclinone und 7-Desoxyanthracyclinone können als Zwischenprodukte zur Herstellung anderer Anthracyclin-Antibiotika angewandt werden, während Auramycin A und B sowie Sulfurmycin A und B antibakterielle Wirkung sowie Antitumorwirkung besitzen.

Die Erfindung betrifft daher auch gegen Tumoren wirksame Mittel, die als Wirkstoff(e) Auramycin A, Auramycin B, Sulfurmycin A und/oder Sulfurmycin B enthalten.

Die biologischen Aktivitäten von Auramycin A und B sowie Sulfurmycin A und B sind:

1. In der folgenden Tabelle 6 sind die minimalen Hemmdosen (MIC) in vitro für Auramycin A und B sowie Sulfurmycin A und B in bezug auf verschiedene Mikroorganismen, bestimmt nach der Agar-Ausstrichmethode, angegeben.

Tabelle 6:

Tabelle 6

Table 6:-

| Strain | | MIC (μg/mℓ) | | | |
|---|---|---|---|---|---|
| | | Auramycin A | Auramycin B | Sulfurmycin A | Sulfurmycin B |
| Bacillus cereus | Ro 179B (1) | 0.39 | 0.2 | 0.2 | 0.39 |
| Bacillus subtilis | IAM 1027 (1) | 3.12 | 0.78 | 0.78 | 0.78 |
| Sarcina lutea | IAM 1009 (1) | 0.1 | 0.05 | 0.1 | 0.1 |
| Staphylococcus aureus 209P | IAM 1011 (1) | 1.56 | 0.39 | 0.78 | 0.78 |
| Staphylococcus aureus 209P Stf | | 0.78 | 0.39 | 0.2 | 0.39 |
| Staphylococcus epidermidis | IFO 12993 (1) | 3.12 | 1.56 | 3.12 | 1.56 |
| Micrococcus flavus | ATCC 10240 (1) | 0.1 | 0.05 | 0.2 | 0.1 |
| Mycobacterium smegmatis | IFO 13167 (1) | 6.25 | 3.13 | 25.0 | 6.25 |
| Escherichia coli K-12 | IAM 1264 (1) | >100 | >100 | >100 | >100 |
| Escherichia coli NIHJ | IFO 12734 (1) | >100 | >100 | >100 | >100 |
| Klebsiella pneumoniae | IFO 3512 (1) | >100 | >100 | >100 | >100 |
| Proteus vulgaris | IAM 1025 (1) | >100 | >100 | >100 | >100 |
| Pseudomonas aeruginosa | IFO 12689 (1) | >100 | >100 | >100 | >100 |
| Serratia marcescens | IFO 12648 (1) | >100 | >100 | >100 | >100 |
| Candida albicans | ATCC 10231 (2) | >100 | >100 | >100 | >100 |
| Candida tropicalis | ATCC 13803 (2) | >100 | >100 | >100 | >100 |
| Saccharomyces cerevisiae | ATCC 9763 (2) | >100 | >100 | >100 | >100 |

(1) Herz infusion -Agar (2) Sabouraud-Dextrose-Agar

2. Akute Toxizität

Die akute intraperitoneale $LD_{50}$ bei Mäusen, beurteilt 72 h nach einer einzelnen Injektion der Antibiotica, beträgt ungefähr 100 mg/kg für Auramycin A, Auramycin B und Sulfurmycin A und 25 bis 50 mg/kg für Sulfurmycin B.

3. Antitumorwirkung

Die erfindungsgemäßen Anthracyclin-Glycoside wurden gegen Leukämie P388 bei Mäusen untersucht. Wenn $CDF_1$ Mäusen $1 \times 10^6$ Zellen von P388 intraperitoneal eingeimpft wurden und jedes der Antibiotica am 1., 5. und 9. Tag intraperitoneal verabreicht wurde, verlängerte sich die Überlebenszeit der behandelten Mäuse wie in der folgenden Tabelle 7 angegeben.

Tabelle 7

| Antibioticum | Dosis (mg/kg·d) | Mittelüberlebenszeit (T/C, %) |
|---|---|---|
| Auramycin A | 15<br>7,5<br>3,75<br>1,88 | 170<br>134<br>129<br>139 |
| Auramycin B | 15<br>7,5<br>3,75<br>1,88 | 196<br>149<br>124<br>113 |
| Sulfurmycin A | 15<br>7,5<br>3,75<br>1,88 | 165<br>144<br>134<br>124 |
| Sulfurmycin B | 15<br>7,5<br>3,75<br>1,88 | 165<br>155<br>124<br>124 |

Wie oben erwähnt, können Auramycin A, Auramycin B, Sulfurmycin A sowie Sulfurmycin B als Arzneimittel gegen Tumoren in Form pharmazeutischer Zubereitungen angewandt werden. Die erfindungsgemäßen Antitumormittel umfassen auch die pharmakologisch verträglichen Salze dieser Verbindungen.

Die pharmazeutischen Zubereitungen enthalten den Wirkstoff zusammen mit einem pharmakologisch verträglichen Träger. Dieser Träger kann ein organisches oder anorganisches inertes Trägermaterial sein, das geeignet ist, für die enterale, percutane oder parenterale Verabreichung, wie z.B. Wasser, Gelatine, Gummiarabicum, Lactose, Stärke, Magnesiumstearat, Talkum, pflanzliche Öle, Polyalkylenglykole, Vaseline u.ä.; die pharmazeutischen Zubereitungen können auch andere therapeutisch wirksame Substanzen als Auramycin A, Auramycin B, Sulfurmycin A und/oder Sulfurmycin B zusätzlich enthalten. Die Arzneimittel können in fester Form (z.B. als Tabletten, Dragees oder Kapseln) oder in flüssiger Form (z.B. als Lösungen, Suspensionen oder Emulsionen) hergestellt werden. Sie können sterilisiert werden und/oder andere Zusätze, wie Konservierungsmittel, Stabilisatoren, Netzmittel, Emulgatoren, Salze zur Änderung des osmotischen Druckes oder Puffer enthalten.

Die Dosis, in der der Wirkstoff verabreicht wird, hängt von der Verabreichungsart, dem Alter, Gewicht und dem Zustand des Patienten und der speziellen zu behandelnden Krankheit ab. Eine typische Dosis für Erwachsene liegt im Bereich von 20 bis 30 mg/Tag bei oraler oder parenteraler Verabreichung, vorzugsweise durch intravenöse Injektion.

Das erfindungsgemäße Verfahren wird durch die folgenden Beispiele näher erläutert.

B e i s p i e l 1

Die von einer Agarschräge abgekratzten Sporen von Streptomyces galilaeus OBB-111 wurden in einen 500 ml-Erlenmeyer-Kolben überführt, enthaltend 100 ml eines sterilisierten Mediums, bestehend aus 20,0 g D-Glukose, 20,0 g lösliche Stärke, 5,0 g S-3 Fleisch (Extrakt) (Ajinomoto Co., Ltd.), 2,5 g Hefeextrakt (Daigo Eiyo-Kagaku Co., Ltd.), 1,0 g Dikaliumhydrogenphosphat, 1,0 g Magnesiumsulfatheptahydrat, 3,0 g Natriumchlorid und 3,0 g Calciumcarbonat, mit Leitungswasser auf 1 l ergänzt. Diese Wachstums-Kultur wurde auf einer Rotationsschüttelvorrichtung mit 180 UpM bei 27°C inkubiert. Nach 72 h wurden 2 ml dieser Kultur in einen 500 ml-Erlenmeyer-Kolben überführt, enthaltend 100 mg steriles Bildungsmedium, bestehend aus 20,0 g D-Glukose, 20,0 g löslicher Stärke, 10,0 g Pharmamedia (Traders Oil Mill Co., USA), 1,0 g Dikaliumhydrogenphosphat, 1,0 g Magnesiumsulfatheptahydrat, 3,0 g Natriumchlorid und 3,0 g Calciumcarbonat, mit Leitungswasser auf 1 l ergänzt. Die Kultur wurde 72 bis 96 h bei 27°C auf einer Rotationsschüttelvorrichtung mit 180 UpM inkubiert.

Zu diesem Zeitpunkt betrug die antibiotische Wirksamkeit des Filtrats bzw. des Mycelextrakts, gemessen mit Hilfe des Papierscheiben-Agar-Diffusionsverfahrens unter Verwendung von Sarcina lutea IMA-1009 als Testmikroorganismus 22 bzw. 20 mm.

B e i s p i e l 2

(a) 600 ml der entsprechend Beispiel 1 erhaltenen Wachstumskultur wurden in einen 50 l-Behälter, enthaltend 30 l steriles Bildungsmedium aus den gleichen Bestandteilen, wie in Beispiel 1 angegeben, und enthaltend zusätzlich 0,1 % Nissan

Disfoam (Nippon Yushi Co., Ltd.) überführt. Die Züchtung wurde bei 27°C unter Rühren mit einem Rührer mit 350 UpM und Belüftung mit 1 Vol./Vol. Medium durchgeführt. Nach ungefähr 90 h wurde die Inkubation beendet.

(b) Das Kulturmedium wurde dann zentrifugiert, das so erhaltene Filtrat und der Filterkuchen wurden getrennt extrahiert. Der Filterkuchen wurde in 15 l Methanol suspendiert, 3 h gerührt und filtriert und der entstandene Filterkuchen erneut mit Methanol extrahiert. 30 l Chloroform und 30 l Wasser wurden zugegeben und mit dem entstandenen Auszug vermischt und die Chloroformschicht abgetrennt. Andererseits wurde das Filtrat mit 60 l eines Lösungsmittelgemisches aus Chloroform und Methanol (1:1) extrahiert und die Chloroformschicht abgetrennt. Die Chloroformauszüge von dem Zellkuchen und dem Filtrat wurden zusammengegeben und auf ein kleines Volumen (50 bis 60 ml) eingedampft. Das Konzentrat wurde mit n-Hexan verdünnt, wobei ein gelber Niederschlag ausfiel, der im Vakuum getrocknet wurde. Man erhielt 4,8 g eines Gemisches aus Auramycin A, Auramycin B, Sulfurmycin A, Sulfurmycin B, Auramycinon, Sulfurmycinon, 7-Desoxyauramycinon und 7-Desoxysulfurmycinon.

(c) Es wurde eine Fraktionierung des oben angegebenen Gemisches durchgeführt. Sephadex LH-20, das 15 h in einem Lösungsmittelgemisch aus Chloroform und Methanol (2:1, Volumen) aufgeschlämmt worden war, wurde in eine Säule mit einer Länge von 50 cm und einem Durchmesser von 5,0 cm gegeben. Das nach dem vorigen Abschnitt erhaltene Gemisch (4,8 g) wurde in 10 ml eines Gemisches aus Chloroform und Methanol (2:1, Volumen) gelöst und auf die Säule aufgegeben. Die Säule wurde mit einem Gemisch aus Chloroform und Methanol (2:1, Volumen) eluiert. Man erhielt zwei deutliche Zonen von Anthracyclinen. Die eine erwies sich bei Dünnschichtchromatographie über Silicagel (Chloroform : Methanol

19:1, Volumen) als Gemisch aus hauptsächlich 7-Desoxyauramycinon und 7-Desoxysulfurmycinon mit geringen Mengen an Auramycinon und Sulfurmycinon. Die Fraktionen, die dieses Gemisch enthielten, wurden im Vakuum zur Trockene eingedampft, wobei man 1,2 g eines gelben Feststoffs erhielt. Es zeigte sich, daß die andere eluierte Zone, die Anthracyclinglycoside enthielt. Die Fraktionen die diese Glycoside enthielten, wurden im Vakuum zur Trockene eingedampft, wobei man 2,1 g eines gelben Feststoffes erhielt.

(d) Der die Auramycine A und B und die Sulfurmycine A und B enthaltende gelbe Feststoff (2,1 g) wurde in einer kleinen Menge Chloroform gelöst und auf eine Säule von 40 cm Länge und 2,5 cm Durchmesser, die mit Silicagel gepackt war, aufgegeben. Nach dem Waschen der Säule mit Chloroform wurde Auramycin B eluiert. Sulfurmycin B und Auramycin A wurden mit einem 98:2 (Volumen) Gemisch aus Chloroform und Methanol extrahiert. Dann wurde Sulfurmycin A mit einem 95:5 (Volumen) Gemisch von Chloroform und Methanol extrahiert. Diese Fraktion war mit einer geringen Menge Auramycin A verunreinigt. Jedesder Eluate wurde im Vakuum zur Trockene eingedampft, wobei man 150 mg rohes Auramycin B, 260 mg eines Gemisches von Auramycin A und Sulfurmycin B bzw.160 mg eines Gemisches von Auramycin A und Sulfurmycin A in Form gelber Pulver erhielt.

(e) Das in Stufe B erhaltene rohe Auramycin B (150 mg) wurde weiter gereinigt durch präparative Flüssigkeits/Gas-Chromatographie. Die Probe wurde in 5 ml eines Lösungsmittelgemisches aus Methylenchlorid und Methanol (99:1, Volumen) gelöst und mit Hilfe eines Gaschromatographen Prep PAK-500/SILICA (Waters Associates, Inc.) chromatographiert. Die mobile Phase war ein 99:1 (Volumen) Gemisch aus Methylenchlorid und Methanol mit einer Fließgeschwindigkeit von

50 ml/min. Die Elution wurde mit Hilfe eines Brechungsindex-Monitors überwacht. Die Fraktionen, die nur Auramycin B enthielten (Dünnschichtchromatographie über Silicagel, Chloroform/Methanol, 29:1, Volumen) wurden gesammelt und im Vakuum auf ein geringes Volumen eingeengt. Die Zugabe von etwas n-Hexan führte zur Ausfällung von 98 mg reinem Auramycin B.

(f) Das in Stufe (d) erhaltene Gemisch aus Sulfurmycin B und Auramycin A (260 mg) wurde entsprechend dem in Stufe (e) beschriebenen Verfahren gereinigt. Die mobile Phase war ein Gemisch aus Methylenchlorid und Methanol (197:3, Volumen) mit einer Fließgeschwindigkeit von 50 ml/min. Die Sulfurmycin B-Fraktionen wurden zuerst eluiert und anschließend die Auramycin A-Fraktionen. Die Fraktionen, enthaltend reines Sulfurmycin B bzw. Auramycin A wurden im Vakuum auf kleine Volumina eingeengt. Nach Zugabe von n-Hexan zu den Konzentraten erhielt man 122mg reines Sulfurmycin B bzw. 54 mg reines Auramycin A.

(g) Das in Stufe (d) erhaltene Gemisch, enthaltend Auramycin A und Sulfurmycin A (160 mg) wurde entsprechend dem in Stufe (e) beschriebenen Verfahren gereinigt. Die mobile Phase war Methylenchlorid zu Methanol (98:2, Volumen) mit einer Fließgeschwindigkeit von 50 ml/min. Die ersten Fraktionen enthielten eine kleine Menge Auramycin A und anschließend folgten die Sulfurmycin A-Fraktionen. Die Fraktionen, enthaltend reines Sulfurmycin A wurden im Vakuum auf ein kleines Volumen eingeengt und ergaben 68 mg reines Sulfurmycin A nach Zugabe einer kleinen Menge n-Hexan.

(h) 1,2 g des gelben Feststoffes, der hauptsächlich aus 7-Desoxyauramycinon und 7-Desoxysulfurmycinon und kleineren Mengen Auramycinon und Sulfurmycinon bestand und in Stufe (c) erhalten worden war, wurden mit Silicagel vermischt und der Säulenchromatographie über Silicagel (Säule 25 x 2,5 cm) unterworfen unter Verwendung eines Gemisches aus Chloroform und n-Hexan (4:1, Volumen) als Eluens. 7-Desoxysulfurmycinon wurde als erstes eluiert und anschließend 7-Desoxyauramycinon, Sulfurmycinon und Auramycinon in dieser Reihenfolge. Die Fraktionen, die nur eine Verbindung enthielten, wurden im Vakuum zur Trockene eingedampft. Man erhielt auf diese Weise 43 mg reines 7-Desoxysulfurmycinon, 68 mg 7-Desoxyauramycinon, 5 mg Sulfurmycinon bzw. 7 mg Auramycinon.

B e i s p i e l 3

Auf analoge Weise wie in Beispiel 2 beschrieben aber unter Verwendung von Weizenkeimen als Stickstoffquelle und unter Verwendung von Streptomyces galilaeus OBB-111-610, erhalten durch Behandlung von Streptomyces galilaeus OBB-111 mit N-Methyl-N'-nitro-N-nitrosoguanidin, erhielt man 153 mg Auramycin B, 171 mg Sulfurmycin B, 137 mg Auramycin A, 149 mg Sulfurmycin A, 55 mg 7-Desoxysulfurmycinon, 78 mg 7-Desoxyauramycinon, 5 mg Sulfurmycinon und 7 mg Auramycinon.

B e i s p i e l 4

In analoger Weise wie in Beispiel 2 beschrieben aber unter Verwendung von Streptomyces galilaeus FR-401 erhielt man 14 mg Auramycin B, 16 mg Sulfurmycin B, 13 mg Auramycin A, 14 mg Sulfurmycin A, 5 mg 7-Desoxysulfurmycinon und 5 mg 7-Desoxyauramycinon.

B e i s p i e l 5

Eine Lösung von 100 mg Auramycin A in 20 ml 0,1 n Salzsäure

wurde 60 min auf 90°C erhitzt . Das Gemisch wurde abgekühlt und mit 40 ml Äthylacetat extrahiert. Die erhaltene Äthylacetatschicht wurde über Natriumsulfat getrocknet und im Vakuum eingedampft. Man erhielt 40 mg eines gelben Pulvers. Beim Umkristallisieren aus n-Hexan/Chloroform erhielt man 30 mg Auramycinon (gelbe Nadeln).

B e i s p i e l 6

Eine Lösung von 100 mg Sulfurmycin A in 20 ml 0,1 n Salzsäure wurde 60 min auf 90°C erhitzt. Das Gemisch wurde abgekühlt und mit 40 ml Äthylacetat extrahiert. Die erhaltene Äthylacetatschicht wurde über Natriumsulfat getrocknet und im Vakuum zu 45 mg eines gelben Pulvers eingedampft. Beim Umkristallisieren aus n-Hexan/Chloroform erhielt man 32 mg Sulfurmycinon (gelbe Nadeln).

B e i s p i e l 7

Eine Lösung von 300 mg eines Gemisches aus Anthracyclinglycosiden, das erhalten worden war nach einem Verfahren, entsprechend Stufe (c) des Beispiels 2, in 20 ml 0,1 n Salzsäure wurde 60 min auf 90°C erwärmt. Das Gemisch wurde abgekühlt und mit 80 ml Äthylacetat extrahiert. Die Äthylacetatschicht wurde über Natriumsulfat getrocknet und im Vakuum eingedampft. Man erhielt 50 mg eines gelben Pulvers, enthaltend Sulfurmycinon und Auramycinon. Das Gemisch der Anthracyclinone wurde mit Silicagel vermischt und der Säulenchromatographie über Silicagel (Säule 30 x 2,5 cm) mit Methylenchlorid unterworfen. Sulfurmycinon wurde zunächst eluiert und anschließend Auramycinon. Die Fraktionen, enthaltend Sulfurmycinon, wurden zusammengegeben und im Vakuum zur Trockene eingedampft. Man erhielt 24 mg Sulfurmycinon in Form eines gelben Pulvers. Die Fraktionen, enthaltend

Auramycinon, wurden ebenfalls zusammengegeben und im Vakuum zur Trockene eingedampft. Man erhielt 11 mg Auramycinon in Form eines gelben Pulvers.

Beispiel 8

Zehn männliche Wister-Ratten wurden durch Abtrennen der Köpfe getötet. Die Lebern wurden entnommen und in einem Glas-Teflon-Homogenisator in 300 ml 0,15 m Kaliumchloridlösung homogenisiert und 10 min mit 9000 g zentrifugiert. Die überstehende Flüssigkeit wurde als Enzymzubereitung verwendet. Ein Gemisch aus 40 ml Enzymzubereitung, 2 ml einer Lösung von Auramycin A (10 mg/ml), 10 mg NADPH und 5 ml 0,1 m Tris-HCl-Puffer (pH 7,8) wurde 45 min bei 37°C unter anaeroben Bedingungen stehen gelassen. Die Reaktion wurde durch Zugabe eines Lösungsmittelgemisches aus Chloroform und Methanol (1:1, Volumen) beendet. Die Chloroformschicht wurde abgetrennt, im Vakuum eingeengt und der Dünnschichtchromatographie (Toluol/Methanol 20:1, Volumen) unterworfen. Die Zone, enthaltend 7-Desoxyauramycinon wurde abgekratzt, mit Chloroform extrahiert und im Vakuum zur Trockene eingedampft. Man erhielt 2,5 mg 7-Desoxyauramycinon.

Beispiel 9

Entsprechend Beispiel 8 erhielt man unter Verwendung von 20 mg Sulfurmycin A 2,3 mg 7-Desoxysulfurmycinon.

Beispiel 10

Enzmyatische Hydrogenolyse von Auramycin A, Auramycin B, Sulfurmycin A und Sulfurmycin B.

Ein 500 ml Erlenmeyer-Kolben, enthaltend 100 ml Wachstumskultur von Streptomyces galilaeus OBB-111, die nach einem Verfahren entsprechend dem in Beispiel 1 beschriebenen erhalten worden war, enthaltend Auramycin A, Auramycin B, Sulfurmycin A und Sulfurmycin B, wurde 12 h bei Raumtemperatur stehen gelassen und dann mit 200 ml eines Lösungsmittelgemisches aus Chloroform und Methanol (1:1, Volumen) extrahiert. Die Chloroformschicht wurde abgetrennt, im Vakuum eingeengt und der Dünnschichtchromatographie (Toluol: Methanol = 20:1, Volumen) unterworfen. Die Zonen, enthaltend 7-Desoxysulfurmycinon und 7-Desoxyauramycinon wurden abgekratzt, mit Chloroform extrahiert und im Vakuum zur Trockene eingedampft. Man erhielt 1,4 mg reines 7-Desoxysulfurmycinon bzw. 1,2 mg reines 7-Desoxyauramycinon.

# P a t e n t a n s p r ü c h e

1) Tetracyclische Verbindung der allgemeinen Formel

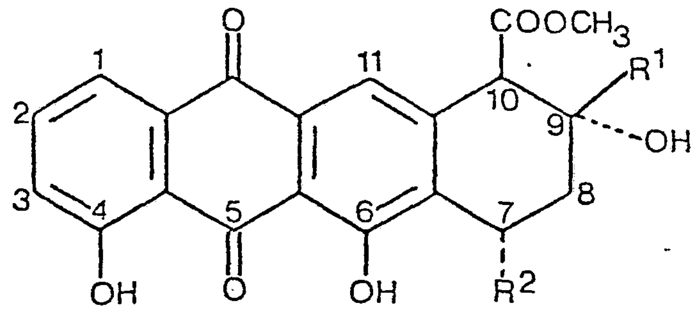

I ,

in der $R^1$ eine Methyl- oder Acetonylgruppe und $R^2$ ein Wasserstoffatom, eine Hydroxylgruppe oder eine Gruppe der Formel

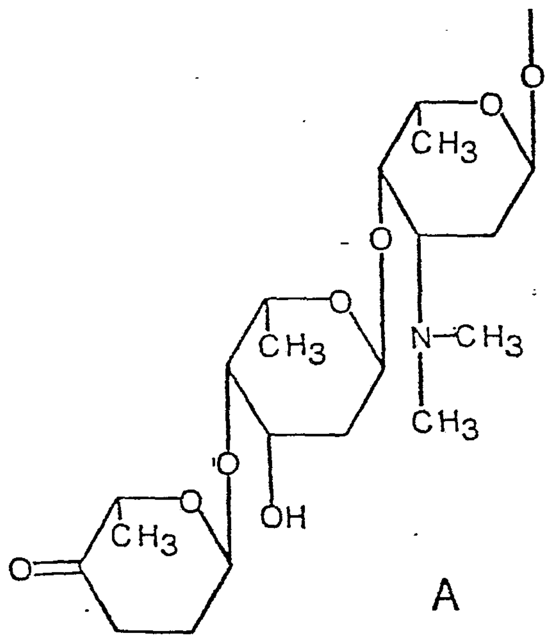

A

oder

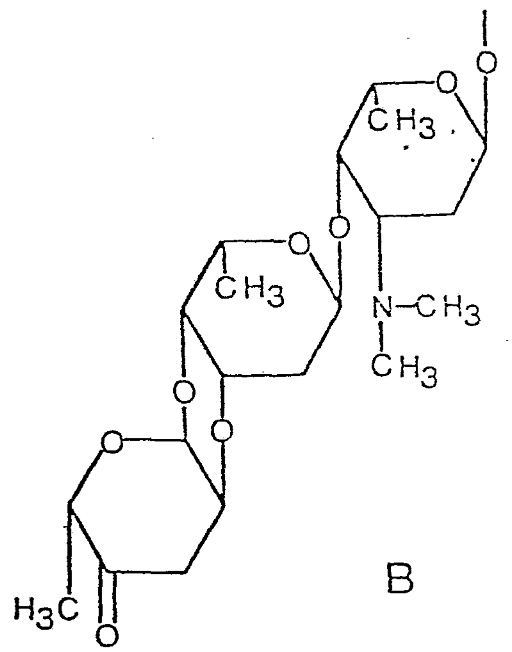

B

bedeuten.

2) Auramycin A nach Anspruch 1.

3) Sulfurmycin A nach Anspruch 1.

4) Auramycin B nach Anspruch 1.

5) Sulfurmycin B nach Anspruch 1.

6) Auramycinon nach Anspruch 1.

7) Sulfurmycinon nach Anspruch 1.

8) 7-Deoxyauramycinon nach Anspruch 1.

9) 7-Deoxysulfurmycinon nach Anspruch 1.

10) Eine Verbindung nach Anspruch 1 der Formel I, wobei $R^2$ der Formel A oder B entspricht, sofern sie als antibakterielles oder Antitumormittel verwendet wird.

11) Verfahren zur Herstellung einer Verbindung der Formel I, dadurch gekennzeichnet,

daß man (a) einen Mikroorganismus der Art Streptomyces galilaeus, der imstande ist, die Verbindungen der Formel I zu bilden, in einem wäßrigen Nährmedium unter aeroben Bedingungen züchtet und die Verbindungen aus der Fermentationsbrühe gewinnt;

(b) eine Verbindung der allgemeinen Formel

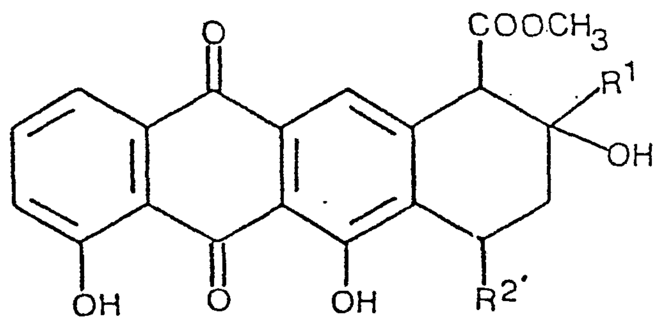

I' ,

in der $R^1$ die in Anspruch 1 angegebene Bedeutung hat und $R^{2'}$ eine Gruppe der Formel A oder B bedeutet, unter sauren Bedingungen hydrolysiert, um die Gruppe $R^{2'}$ in eine Hydroxylgruppe umzuwandeln oder

(c) die Zuckergruppe von einer Verbindung der Formel I' mit Hilfe eines Katalysators oder eines Enzyms abspaltet, um die Gruppe $R^{2'}$ der Formel I' durch ein Wasserstoffatom zu ersetzen.

12) Verfahren nach Anspruch 11, dadurch g e k e n n z e i c h n e t , daß man als Mikroorganismus in Stufe (a) Streptomyces galilaeus OBB-111, OBB-111-610 oder FR-401 verwendet.

13) Verfahren nach Anspruch 11, dadurch g e k e n n z e i c h n e t , daß man die saure Hydrolyse der Stufe (b) in Gegenwart einer Mineralsäure bei einer Temperatur von 0°C bis zur Rückflußtemperatur des Hydrolysegemisches durchführt.

14) Verfahren nach Anspruch 11, dadurch g e k e n n z e i c h n e t , daß man als Katalysator in Stufe (c) Palladium, Platin, Rhodium oder Aktivnickel verwendet.

15) Verfahren nach Anspruch 11, dadurch g e k e n n z e i c h n e t , daß man als Enzym in Stufe (c) ein solches verwendet, das erhalten worden ist aus Tierleberhomogenaten oder von Zellen von Anthracyclin-Antibiotikabildenden Mikroorganismen.

16) Arzneimittel mit antibakterieller und Antitumorwirkung auf der Basis von Auramycin A, Auramycin B, Sulfurmycin A und/oder Sulfurmycin B.

17) Verwendung von Auramycin A, Auramycin B, Sulfurmycin A und Sulfurmycin B als antibakterielle oder Antitumormittel.

***

Patentansprüche [redacted]

1) Verfahren zur Herstellung tetracyclischer Verbindungen der allgemeinen Formel

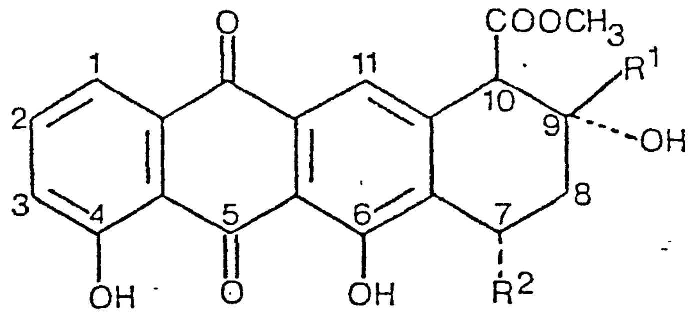

I ,

in der $R^1$ eine Methyl- oder Acetonylgruppe und $R^2$ ein Wasserstoffatom, eine Hydroxylgruppe oder eine Gruppe der Formel

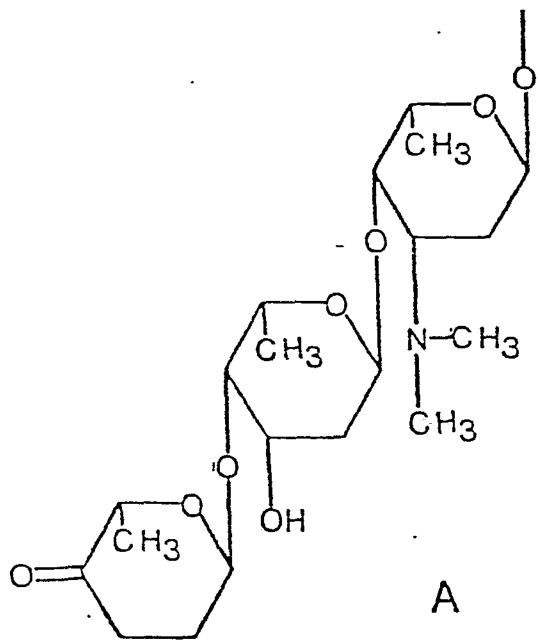

A

oder

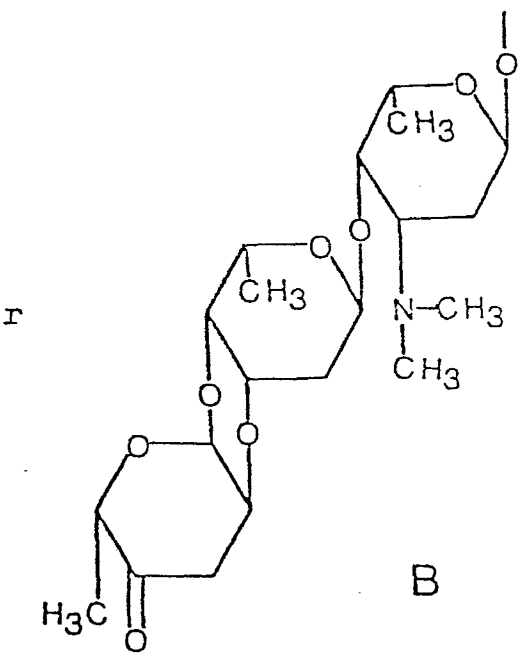

B

bedeuten, dadurch gekennzeichnet,

daß man (a) einen Mikroorganismus der Art Streptomyces galilaeus, der imstande ist, die Verbindungen der Formel I zu bilden, in einem wäßrigen Nährmedium unter aeroben Bedingungen züchtet und die Verbindungen aus der Fermentationsbrühe gewinnt;

(b) eine Verbindung der allgemeinen Formel

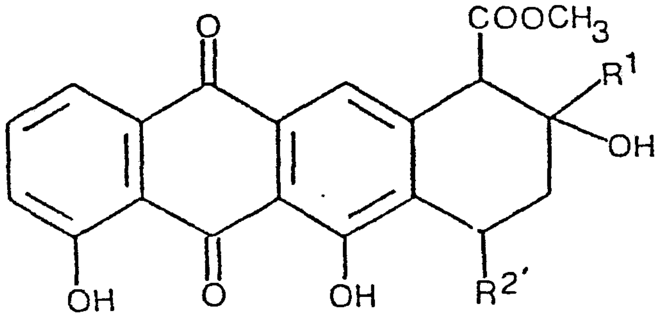

I' ,

in der $R^1$ die in Anspruch 1 angegebene Bedeutung hat und $R^{2'}$ eine Gruppe der Formel A oder B bedeutet, unter sauren Bedingungen hydrolysiert, um die Gruppe $R^{2'}$ in eine Hydroxylgruppe umzuwandeln oder

(c) die Zuckergruppe von einer Verbindung der Formel I' mit Hilfe eines Katalysators oder eines Enzyms abspaltet, um die Gruppe $R^{2'}$ der Formel I' durch ein Wasserstoffatom zu ersetzen.

2) Verfahren nach Anspruch 1, dadurch gekennzeichnet, daß man als Mikroorganismus in Stufe (a) Streptomyces galilaeus OBB-111, OBB-111-610 oder FR-401 verwendet.

3) Verfahren nach Anspruch 1, dadurch gekennzeichnet, daß man die saure Hydrolyse der Stufe (b) in Gegenwart einer Mineralsäure bei einer Temperatur von 0°C bis zur Rückflußtemperatur des Hydrolysegemisches durchführt.

4) Verfahren nach Anspruch 1, dadurch gekennzeichnet, daß man als Katalysator in Stufe (c) Palladium, Platin, Rhodium oder Aktivnickel verwendet.

5) Verfahren nach Anspruch 1, dadurch gekennzeichnet, daß man als Enzym in Stufe (c) ein solches verwendet, das erhalten worden ist aus Tierleberhomogenaten oder von Zellen von Anthracyclin-Antibiotika-bildenden Mikroorganismen.

***

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung 0019302

EP 80102827.5

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | DE - A1 - 2 715 255 (ZAIDAN)<br>+ Seiten 1-15 +<br>-- | 1,11a,<br>16 | C 07 C 69/95<br>C 07 H 15/24<br>C 12 P 19/56<br>C 12 P 29/00<br>A 61 K 31/71 |
| | CH - A - 374 146 (CIBA)<br>+ Gesamt +<br>---- | 1,11a,<br>12,16 | |
| | | | **RECHERCHIERTE SACHGEBIETE (Int Cl.³)** |
| | | | C 07 C 69/00<br>C 07 H<br>C 12 P<br>A 61 K 31/00 |

**UNVOLLSTÄNDIGE RECHERCHE**

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-16

Unvollständig recherchierte Patentansprüche: -

Nicht recherchierte Patentansprüche: 17

Grund für die Beschränkung der Recherche:

Verfahren zur therapeutischen Behandlung des menschlichen oder tierischen Körpers (Art. 52(4) EPÜ)

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| WIEN | 05-08-1980 | JANISCH |